Europäisches Patentamt

**European Patent Office**

**Office européen des brevets**

(19)

(11) Publication number: **0 017 460**

**A1**

(12) **EUROPEAN PATENT APPLICATION**

(21) Application number: **80301003.2**

(22) Date of filing: **31.03.80**

(51) Int. Cl.³: **G 01 N 33/54**
**//(C12Q1/70)**

(30) Priority: **02.04.79 US 26173**

(43) Date of publication of application:
**15.10.80 Bulletin 80/21**

(84) Designated Contracting States:
**BE CH DE FR GB IT LU NL SE**

(71) Applicant: **Research Corporation**
**405 Lexington Avenue**
**New York, N.Y. 10017(US)**

(72) Inventor: **Caldwell, Harlan D.**
**2138 Wilmington Drive**
**Walnut Creek California 94956(US)**

(72) Inventor: **Kuo, Cho-Chou**
**6531 29th Avenue NE**
**Seattle Washington, 98115(US)**

(74) Representative: **Harrison, David Christopher et al,**
**MEWBURN ELLIS & CO 70 & 72 Chancery Lane**
**London WC2A 1AD(GB)**

(54) **Immunofluorescent test method and substance for use therein.**

(57) *Chlamydia trachomatis* organisms are responsibe for a number of human ocular/genital diseases. In order to test for infection by such organisms, reticulated body derived from any immunotype strain of *C. trachomatis* is utilized in a micro-immunofluorescence testing, which allows the detection of antibody to *C. trachomatis* regardless of strain or immunotype. If anti-human IgM is utilized in the fluorescent conjugate it can be determined if the antibody is derived from a human source of active infection.

EP 0 017 460 A1

IMMUNOFLUORESCENT TEST METHOD AND SUBSTANCE FOR USE THEREIN

This invention relates to immunofluorescence test methods and to a substance for use therein. Chlamydia trachomatis organisms are the etiological agents for a number of human ocular-genital diseases, such as ocular trachoma, lymphogranuloma venereum (LGV) and non-gonococcal urethritis (NGU). The most important of these diseases in the United States is NGU. The annual incidence of NGU exceeds that of gonorrhea and it is estimated that there occur more than 2,000,000 cases of NGU yearly in the United States alone.

The most sensitive and specific serology of Chlamydia trachomatis has been the indirect micro-immunofluorescence antibody test (micro-IF) of Wang and Grayston (J. Infect. Dis 130 388 (1974)), currently used in several major chlamydial research laboratories throughout the world. However, due to the difficulty of the test, it has not yet been incorporated as a diagnostic procedure in general clinical laboratories. The standard micro-IF test requires inclusion of 15 C. trachomatis immunotype elementary body (EB) antigens. Others have used a single immunotype strain with broad cross reactivity in the micro-IF (Thomas et al J. Clin. Microbiol. 4:6 (1976)). This latter approach has practical value because in many instances information
of specific immunotypes is not required for diagnosis
and treatment of chlamydial infections. Unfortunately,
no C. trachomatis strain cross-reacts a hundred per
cent with all 15 C. trachomatis immunotypes. Therefore,
the usage of a single antigen has compromised the
simplicity of the test in return for reduction in
sensitivity.

A different approach to this problem was taken by
Caldwell et al, U. S. Patent No. 4,118,469 who isolated
a single antigen common to all strains of C. trachomatis.
This is used in the radioimmunoassay method.

In the micro-IF test, the use of a group specific
antigen would make use of an antibody's ability to cross-
react with a broadly reacting antigen. Technically,
the micro-IF requires the intact morphology or structure
of the organism for identification of the immunofluorescent reaction. Therefore, any procedure which alters
or destroys the morphology of chlamydia cannot be
used. However, if an intact form of the organism
could be used which would be capable of providing
broad cross-reactivity to antisera of all the immunotypes, this antigenic form should provide the sensitivity needed from a single test antigen.

Christophersen and Manire (J. Immunol. 103, 1085 (1969)),
working with C. Psittaci observed that the two developmental
forms of chlamydiae, i.e., reticulated bodies (RBs)
and elemental bodies (EBs) have distinct characteristics.
RB is non-infectious and does not contain the characteristic chlamydial "toxins", EB is infectious and contains
the "toxins". Wang observed an exact correlation
of the specificity of the mouse toxicity prevention
test (Wang, et al. 1963. Classification of trachoma
virus strains by protection of mice from toxic death.
J. Immunol. 90: 849-856.) and the micro-IF (Wang,
et al. 1971. A micro immunofluorescence method.
Study of antibody response to TRIC organisms in mice,
p. 273-288. In R.L. Nichols (3d.), Trachoma and
related disorders caused by chlamydial agents. Excerpta
Medica, Amsterdam.). This observation indicated that
the type-specific antigens might be the same as the
"toxins" in the more mature EB form.

It has been found that RB used as antigen in the micro-
IF test cross-reacted with all C. trachomatis immunotypes
and psittacosis, indicating species as well as group
reactivity. The test using the RB antigen was as
sensitive as the test using multiple EB antigens in
C. trachomatis serology, resulting in a further simplification of the micro-IF test.  Positive sera can be tested
for type specificity by the standard micro-IF test
later, if needed.  Furthermore, the larger size of
the RB makes for easier reading in the fluorescent
antibody microscope.  In addition, the antigen can
be stored at $4^{\circ}$C for at least 1 year, making the RB
antigen extremely useful in the serological screening
of sera for chlamydial antibodies even in laboratories
not specialized in chlamydia research.

By utilizing IgM or IgG antihuman antibodies the test
is further refined since IgM antibodies will only
react in the case of inactive infection while IgG
antibody will react when any antibody to the RB antigen was present in the serum.

The C. trachomatis strains utilized in the preparation
of the reticulated body which is the subject of the
present invention are grown in a tissue culture suitably in HeLa 229 monolayers in accordance with the
method of Kuo, et al (Growth of trachoma organisms
in HeLa 229 cell culture, p. 328-336. In D. Hobson
and K.K.  Holmes (ed.), Nongonococcal urethritis and
related infections.  ASM Publications, Washington
DC.)  In this method, one-day old HeLa cell monolayers
are inoculated with about 10 inclusion forming units/
cell of organisms to insure 100% infectivity of the cells. The infected cells are harvested in the early
stages of growth to assure a substantial predominance
of RB over the EB form. Such harvesting should suitably be done between 24 and 28 hours post infection.
The cells are then disrupted for a very brief period
suitably about 30 seconds by sonication and subjected
to one cycle of differential centrifugation. The
thus obtained pellet is then layered on a suitable
high-density medium suitably renografin preferably
35% renographin and centrifuged. This centrifugation
serves to retain low molecular weight components. The resultant pellet containing the desired high molecular
weight component is resuspended in a suitable buffer
and layered on linear gradient renografin and recentrifuged. The major band appearing in the middle
of the 35% through 60% gradient is collected diluted
with saline, recentrifuged and in the preferred embodiment of this invention, stabilized. Such stabilization
may be carried out by treatment with formaldehyde
suitably formalin or with gluteraldehyde both in
saline solution. The thus stabilized RB bodies may
be stored for a substantial period of time at reduced
temperatures suitably at about 4°C.

The micro-IF test utilizing the RB is substantially
that disclosed by Wang (Wang, et al, 1963. Classification of trachoma virus strains by protection of mice from toxic death. J. Immunol. 90:849-856.) as improved by Wang and Grayston (J. Infect. Dis. 130:388 at 389.). In this test there is prepared a substrate, suitably a clean microscope slide. A guidance template indicating  test loci is placed under the slide and samples of the antigen (that is to say the RB samples) are placed on the slide by suitable means for example a pen point on those areas indicated by the template. The slides are then air-dried and the antigen affixed upon the substrate suitably by immersion in acetone for ten to fifteen minutes at ambient temperatures.

The sample material suspected of containing antibodies to C. trachomatis is then subjected to serial dilution in the usual manner. These sources may be animal sera suitably mouse or rabbit sera or, in the expected application of this test, may be human sources suitably human sera, tears or endocervical secretions. The test samples are placed upon the antigen dots with a bacteriologic loop. The slides are incubated in a moist environment suitably at about 37$^\circ$C for about 30 minutes and the slides washed gently preferably with phosphate buffered saline to remove the unreacted suspected antibody source material.

The substrate is then treated with anti (source materials species) antibodies coupled with the fluorescent materials thus where the antibody source material is the mouse then there can be used for example, goat anti-mouse gammaglobulin conjugated with, for example, fluorescein isothiocyanate. Where the source material is human there may be utilized similarly coupled goat anti-human gammaglobulin. It will be clear to those skilled in the art what type of anti-species antibodies should be utilized.

In a particularly preferred modification of the invention, where the antibody source material is human, there is utilized a fluorescent conjugate of anti-human IgM antibody and anti-human IgG antibody. Since IgM is only present in an active infection this mode of proceeding provides a method of determining whether the antibody source material comes from an active or a passive infection.

The conjugate solution is applied to the slides with a bacteriologic loop in the manner described above and the substrate incubated, rinsed, washed and air-dried. The slides are then examined under a source of UV light. The presence of fluorescence indicating the presence of antibody to C. trachomatis in the test samples.

## EXPERIMENTAL METHODS

<u>C. trachomatis strains.</u>

<u>C. trachomatis</u> strains used for preparing RB were B/TW-5/OT, C/TW-3/OT, and $L_2$/434/Bu. The strains used for the micro-IF EB slide antigens were <u>C. trachomatis</u> A/G-17/OT, B/TW-5/OT, Ba/AP-2/OT, C/TW-3/OT, D/UW-3/Cx, E/UW-5/Cs, F/UW-55/Ur, G/UW-57/Cx, H/UW-43/Cx, I/UW-12/Ur, J/UW-35/Cx, K/UW-31/Cs, $L_1$/440/Bu, $L_2$/434/Bu, $L_3$/404/Bu, mouse pneumonitis, Nigg (MoPn), and <u>C. psittaci</u> meningopneumonitis, Cal-10 (Mn). These strains have been described:

Kuo, C.C., S.P. Wang, J.T. Grayston and E.R. Alexander. 1974 TRIC type K, a new immunologic type of <u>Chlamydia trachomatis</u>. J. Immunol. <u>113</u>:591-596.

Wang, S.P. and J.T. Grayston. 1971. Classification of TRIC and related strains with micro immunofluorescence, p. 305-321. <u>In</u> R.L. Nichols (ed.), Trachoma and related disorders caused by chlamydial agents. Excerpta Medica, Amsterdam.

Wang, S.P. and J.T. Grayston. 1974. <u>Chlamydia trachomatis</u> immunotype J. J. Immunol. <u>115</u>:1711-1716.

Wang, S.P., J.T. Grayston and J.L. Gale. 1973. Three new immunological types of trachoma-inclusion conjunctivitis organisms. J. Immunol. <u>110</u>:837-879.

All were grown in HeLa 229 cell culture. (Kuo, C.C., S.P. Wang and J.T. Grayston. 1977. Growth of trachoma organisms in HeLa 229 cell culture, p. 328-336. <u>In</u> D. Hobson and K.K. Holmes (ed.), Nongonococcal urethritis and related infections. ASM Publications,

Washington, D.C.

Designation of Equivalent (Cross Reactive) Immunotypes:

| Representative strains indicated in the present application | Strains at ATCC (Dr. D.A. Stevens) | | Strains available at WHO - Trachoma Reference Laboratory |
|---|---|---|---|
| A/G-17/OT | A/HAR-13(or Eg-2)/OT | VR 571 | A/HAR-1(or SA-1)/OT |
| B/TW-5/OT | B/HAR-36/OT | VR 573 | B/TW-1/OT |
| Ba/AP-2/OT | Ba/AP-2/OT | VR 347 | Ba/AP-2/OT |
| C/TW-3/OT | C/TW-3/OT | VR 578 | C/TW-3/OT |
| | C/PK-2/OT | VR 576 | |
| D/UW-3/Cx | D/UW-3/Cx | VR 885 | D/IC-Cal-8/ON |
| E/UW-5/Cx | E/Bour/OT | VR 348 | E/Bour/OT |
| F/UW-94/Ur | F/IC-Cal-3/ON | VR 346 | F/IC-Cal-3/ON |
| G/UW-57/Cx | G/UW-57/Cx | VR 878 | G/392/OC |
| H/UW-43/Cx | H/UW-43/Cx | VR 879 | H/471/Cx |
| I/UW-12/Ur | I/UW-12/Ur | VR 880 | I/870/OC |
| J/UW-36/Cx | J/UW-36/Cx | VR 886 | J/UW-36/Cx |
| K/UW-31/Cx | K/UW-31/Cx | VR 887 | K/UW-31/Cx |
| $L_1$/440/Bubo | | | $L_1$/440/Bubo |
| $L_2$/434/Bubo | $L_2$/JH | VR 121 | $L_2$/434/Bubo |
| $L_3$/404/Bubo | | | $L_3$/404/Bubo |

The strain designation is indicated by: Immunotype (A,B,C, ...$L_3$)/Strain-number/Origi

The abbreviations are: OT-ocular trachoma, ON-ophthalmia neoratorum, OC-ocular conjunctivitis, Cx-cervix, Ur-urethra, Bubo-lymph nodes of LGV patient.

Strains of the same immunotype are interchangable in the micro-IF test.

\* c/o Dr. J. Schachter, University of California, San Francisco Medical Center, San Francisco, California 94122, U.S.A.

Growth and preparation of RB.

C. trachomatis strains TW-3 and TW-5 were grown in
HeLa 229 cell monolayers (Kuo, C.C., S.P. Wang and
J.T. Grayston. 1977. Growth of trachoma organisms
in HeLa 229 cell culture, p. 328-336. In D. Hobson
and K.K. Holmes (ed.), Nongonococcal urethritis and
related infections. ASM Publications, Washington,
D.C.) and LGV $L_2$/434/Bu in L cell suspension culture
(Caldwell, H.D., C.C. Kuo and G.E. Kenny. 1975. Antigenic analysis of Chlamydiae by two-dimensional immuno-
electrophoresis. I. Antigenic heterogeneity between
C. trachomatis and C. psittaci. J. Immunol. 115:963-
967.)

One-day-old HeLa cell monolayers in 32 oz. prescription
bottles were inoculated with about 10 inclusion forming
units/cell or organisms to assure 100% infectivity
of the cells.  Infected cells were harvested 28 h
post infection.  Cells were disrupted by sonication
for 30 s with a medium probe at 60 intensity (Biosonik
III, Bronwill Scientific, Rochester, NY).  After one
cycle of differential centrifugation (500 xg/10 min
and 30,000 xg/30 min), the pellet was layered on 35%
renografin (methylglucamine diatrizoate, 76% for infection, Squibb and Sons, NY) and centrifuged at 22,000
xg for 1 h in a Spinco Model L ultracentrifuge using
a horizontal rotor SW 25.1 (Beckman Instruments Inc.,
Palo Alto, CA).  The resulting pellet was resuspended in
buffer and layered on a 35%-60% renografin linear gradient and centrifuged at 42,000 xg for 1.5 h. One major band appeared in the middle of the gradient. This band consisted predominantly of RB. The RB band was collected with a capillary pipette, and diluted with 10 mM TES saline, pH 7.0, and pelleted at 30,000 xg for 30 min. RBs were fixed immediately by resuspending with 6% formalin in 10 mM TES saline, centrifuged, resuspended in 0.02% formalin in 10mM TES saline, and stored in a 4°C refrigerator for use. Eighteen culture bottles were usually inoculated per preparation and the final product resuspended in 1 ml of 0.02% formalin. In the above procedure glutaraldehyde may be used in place of formaldehyde.

C. trachomatis serology.

The serology was performed using the micro-IF test as described by Wang (J. Infect. Dis. 130:388 (1974)). RB antigens were placed on the microscope slide along with the EB antigens of the particular immunotypes which were to be compared. Two-fold serial serum dilutions were used. Tears and endocervical secretions were tested in the same way as serum. The dilution factors of the eluate were 1:10 for tear and 1:15 for endocervical secretions. Fluorescein conjugated goat anti-human immunoglobulins IgMAG (combined), igM (μ-chain specific), and IgG (γ-chain specific), were obtained from Hyland Laboratories, Los Angeles, CA. The highest serum dilution which gave a definite fluorescence was taken as endpoint. Titers of 32 or greater with mouse serum and 8 or greater with

0017460

human serum were regarded as positive. Duplicate slides were run each time. Results which agreed in the duplicate slides were recorded. Where duplicate slides did not agree, the slide showing the higher titer for EB was recorded as the result for the test.

Sensitivity and specificity of RB in testing mouse antisera.

The RBs of types C/TW-3/OT, B/TW-5/OT, and $L_2$/434/Bu were used as test antigens against mouse antisera of 15 C. trachomatis immunotypes.

Table 1

Serological reactions of reticulate body (RB) antigens of
3 *C. trachomatis* strains (C/TW-3/OT, $L_2$/434/Bu, B/TW-5/CT)
against *C. trachomatis* type specific mouse sera as compared
to that of homotypic elementary body (EB) antigens

| C. trachomatis immunotypes | Antibody titers against homotypic $EB^a$ | Titer difference between RB and homotypic EB antigens (number of 2-fold dilutions)[b] | | |
|---|---|---|---|---|
| | | EB – TW-3 RB | EB – 434 RB | EB – TW-5 RB |
| **C group** | | | | |
| C | 256 | 0,0 | 1,2 | 4,4 |
| J | 256 | 1,0 | 4,2 | |
| A | 256 | 0,1 | 4,4 | |
| H | 128 | 0,0 | 1,1 | |
| I | 256 | 2,2 | 4,4 | |
| K | 256 | 1,1 | 3,3 | |
| $L_3$ | 1,024 | 0,0 | 0,0 | |
| **B group** | | | | |
| B | 256 | 3,2 | 0,2 | 1,1 |
| Ba | 256 | 2,1 | 3,0 | |
| D | 256 | 1,1 | 0,2 | |
| E | 256 | 1,0 | -1,0 | |
| $L_1$ | 128 | 2,1 | 0,1 | |
| $L_2$ | 2,048 | 0,0 | 0,0 | |
| G | 512 | 2,3 | 0,0 | |
| F | 128 | 1,1 | 0,2 | |

*a.* Titers of less than 1:16 were regarded as negative. Three
normal mouse sera were negative to both RB's and EB's.

*b.* Results of duplicate tests with a type specific serum; under-
line means homologous system; blanks mean not tested.

These 3 strains represent two major immune complexes,
i.e., B and C complexes (Wang, S.P., J.T. Grayston,
C.C. Kuo, E.R. Alexander and K.K. Holmes. 1977. Sero-
diagnosis of Chlamydia trachomatis infection with
the micro immunofluorescence test, p. 237-238. In
D. Hobson and K.K. Holmes (ed.), Nongonococcal urethritis
and related infections. ASM Publications, Washington,
D.C.) as well as two groups of biologically distinct
organisms, i.e., trachoma and LGV.  C/TW-3/OT RB reacted
with the same sensitivity to C complex antisera, except
for types I and K antisera which were 2 to 3 two-fold
dilutions less sensitive than the EB.  However, the
C/TW-3/OT RB reaction to the B complex sera was one
to three two-fold dilutions less sensitive than the
EB, except for $L_2$ which exhibited comparable sensitivity.
$L_2$/434/Bu RB in general showed comparable sensitivity
to the B complex but was from one to four two-fold
dilutions less sensitive to the C complex, with the
exception of $L_3$ which had the same sensitivity.  B/TW-
5/OT was one two-fold dilutions less sensitive than
the homotypic EB, but was 4 two-fold dilutions less
sensitive in detecting type C antibody.  It was not
tested further with other immunotypes for this reason.
C/TW-3/OT was also tested against MoPn and Mn antisera.
The results were not conclusive due to low antiserum
titers.  Since C/TW-3/OT RB gave better results in
cross-reacting with all immunotypes than the two other
RB preparations it was used for the remaining studies.

0017460

C/TW-3/OT RB harvested at 28 h was contaminated with
a trace amount of EB.  The transition of RB to EB
in the developmental cycle is a continuous process
not only in size but also in density.  These two populations could not be separated by density gradient centrifugation.  However, contamination with trace EB
did not interfere with micro-IF serology.

Use of a single RB antigen (C/TW-3/OT) in the micro-
IF is a useful, practical, simple, and sensitive serological test for C. trachomatis.

Table 2

Serological reactions of *C. trachomatis* C/TW-3/OT
reticulate body (RB) against human sera as compared
to homotypic elementary body (EB) antigens in the
micro-immunofluorescence antibody test

| *C. trachomatis* immunotype sera | Range of antibody titer to homotypic EB[a] | Titer difference between homotypic EB and TW-3 RB antigens (no. of 2-fold serial dilutions in EB minus RB)[b] |
|---|---|---|
| **C group** | | |
| CJ | 32–512 | 0,0,0,0,0,0 |
| A | 16–256 | 1,0,0 |
| H | 32–1024 | 0,0,0 |
| I | 32–256 | 0,0,>4 |
| K | 32–1024 | 0,0,0,0,0,0,>2,>4 |
| $L_3$ | 512–1024 | 0,0 |
| **B group** | | |
| B | 16–128 | 0,0,0,1 |
| ED | 32–1024 | -1,0,0,0,0,0,0,0,1 |
| $L_1$ | 64–512 | -2,0 |
| $L_2$ | 128–512 | 0,0,0 |
| GF | 32–256 | 0,0,0,0,0,0,0,0,0 |

a. Reciprocal of 2-fold serum dilutions.
b. Results of test on one serum from each person;
underline indicates isolation positive cases.

Antibody to RB in control sera.

Fifty-one different sera from University of Washington staff and students not connected with trachoma research were used as control sera. Five (9.8%) showed titers of 1:8 or greater, four had titers of 1:18 to 1:1aa6, one had titer of 1:64. All five had antibodies to EB which consisted of immunotypes, one each of A, CJ, GF and two B.

Reaction of RB to psittacosis patients sera.

Twelve sera from former psittacosis patients were tested against C/TW-3/OT RB: three were negative, four equivocal and five positive. Under micro-IF the four equivocal results appeared as untypical RB with the rings appearing as slightly undulating circles, resulting in a "patchy" appearance rather than sharp, clear, round cocci. The titers of the five positive cases were 8, 16, 64, 64 and 64. Positive reaction to RB did not correlate with psittacosis complement fixation titers.

Detection of antibodies in tears and endocervical secretions with RB.

Thirty-five tear specimens and 17 endocervical secretions were tested against C/TW-3/OT RB as well as against multiple EB antigens in routine micro-IF serology. Three 2-fold dilutions were used (1:10, 1:20 and 1:40) for tears and three 4-fold dilutions (1:15, 1:60 and 1:240) for endocervical secretions. EBs were found to detect antibodies in tears better than C/TW-3/OT RB (Table 3): 66% positive for EB and 60% positive for RB. Fifteen of twenty-three (65%) positive tear specimens were type C, one type GF and the rest type B. Nine tear specimens had higher titers to EB than to RB; two with two-tube difference and 7 with one-tube difference. Two specimens were EB positive and RB negative. The remaining 12 had equal titers to EB and RB.

However, with endocervical secretions RB showed more positive reactions than EB, 59% vs. 53% respectively. Most of the titers were the same for RB and EB. Only one case showed a 1 tube higher titer for the EB with the RB negative. Three cases showed a 1 tube higher titer for RB with EB negative in one of these 3 cases. The immunotypes found were four ED and six GF. The overall relative sensitivity of RB to EB for both tears and endocervical secretions was 97%.

Table 3

Comparative sensitivity of antibody detection in tears and endocervical secretion by *C. trachomatis* C/TW-3/OT reticulate body (RB) and multiple elementary body (EB) antigens in the micro-immunofluorescence antibody test (micro-IF) using anti-human immunoglobulin (IgMAG) conjugate

| Specimens[a] | No. tested | Micro-IF antibodies to | | | | | | Relative sensitivity (%) (RB ÷ EB) x 100 |
|---|---|---|---|---|---|---|---|---|
| | | multiple EB antigens[b] | | | TW-3 RB antigen | | | |
| | | + | − | %(+) | + | − | %(+) | |
| Tear | 35 | 23 | 12 | 66 | 21 | 14 | 60 | 91 |
| Endocervical secretion (Cx) | 17 | 9 | 8 | 53 | 10 | 7 | 59 | 111 |
| Tear + Cx | 52 | 32 | 20 | 62 | 31 | 21 | 60 | 97 |

a. Tears were obtained from trachoma patients and their family members and endocervical secretions from the patients attending a venereal disease clinic. Initial dilution factor of tear was 1:10 and Cx was 1:15.

b. Included A,B,CJ,ED,GF,H,I,K immunotypes.

Detection of IgM and IgG antibodies and routine serology
of Venereal Disease patients.

Micro-IF with C/TW-3/OT RB was incorporated in the
routine serology of VD patients. Sixty of these sera
were also simultaneously tested for both IgM and IgG
antibodies (Table 4).

Table 4

Comparative sensitivity of use of *C. trachomatis* C/TW-3/OT
reticulate body and multiple elementary body antigens in
the detection of serum IgM and IgG antibodies in
nongonococcal urethritis patients and their
sexual contacts with the micro-immunofluorescence
antibody test (micro-IF)

| Antibody class | Serological reaction | Micro-IF antibodies to | |
|---|---|---|---|
| | | TW-3 RB antigen | multiple EB antigens[a] |
| IgM | +[b] | 4[c] | 4 |
| | − | 56 | 56 |
| IgG | + | 54 | 54 |
| | − | 6 | 6 |
| Total | | 60 | 60 |

a. Included A,B,CJ,ED,GF,H,I,K immunotypes.
b. Titers of 1:8 or greater.
c. Number of sera.

The presence of IgM antibody indicates the presence
of an active infection.

The routine serological test used three 4-fold serum dilutions, 1:8, 1:32 and 1:128.  (Table 5)

Table 5

Comparative sensitivity of use of *C. trachomatis* C/TW-3/OT reticulate body and multiple elementary body antigens in the detection of serum antibodies in nongonococcal urethritis patients and their sexual contacts with the micro-immunofluorescence antibody test (micro-IF)

| Serological reaction | Micro-IF antibodies to | |
|---|---|---|
| | TW-3 RB antigen | multiple EB antigens[a] |
| positive[b] | 78[c] (80%) | 68 (70%) |
| negative | 19 (20%) | 29 (30%) |
| Total[d] | 97(100%) | 97(100%) |

a. Included A,B,CJ,ED,GF,H,I,K immunotypes.
b. Titers of 1:8 or greater.
c. Number of sera.
d. From a total of 63 patients (49 males & 14 females). Fifteen were isolation positive for *C. trachomatis*.

CLAIMS:

1. An immunofluorescent method for the serological testing of sources suspected to contain antibodies characterised in that for testing for antibodies to <u>Chlamydia trachomatis</u> there is employed stabilized reticulated body derived from <u>C. trachomatis</u> as the antigen in order to detect the formation of an antigen-antibody complex.

2. An immunofluorescent method according to claim 1 for the serological testing of sources suspected to contain antibodies to <u>Chlamydia trachomatis</u> comprising the steps of:

a) contacting an inert substrate with a predetermined amount of reticulated body <u>C. trachomatis</u> previously stabilized with formaldehyde

b) fixing a portion of said reticulated body to said substrate

c) removing the non-fixed reticulated body from the substrate

d) contacting said substrate having said reticulated body fixed thereto with a sample of source material suspected to contain antibody to <u>C. trachomatis</u>

e) removing the non-reacted antibody source material

f) contacting the thus formed reticulated body antigen/antibody combination with an anti(source material species) antibody having a fluorescent moiety conjugated therewith

g) removing the unreacted anti-(source material species) antibody

h) examining the substrate under ultraviolet light wherein the presence of fluorescence indicates the presence of C. trachomatis antibodies in the suspected antibody source material.

3.  A method of claim 2 characterized in that the source material is selected from the group consisting of human tears, human endocervical secretion or human serum.

4.  A method of claim 3 characterized in that the anti-(source material species) antibody is antihuman IgG antibody or antihuman IgM antibody.

5.  A method of any one of claims 2-4 characterized in that the reticulated body antigen is derived from at least one strain having an antigenic reaction to antibodies to at least one of the strains designated in WHO reference as:

ASA-1/OT
B/TW1
C/TW8/OT
D/IC-Ca180N
E/Bour/OT
F/IC-Cal-30N
G/392/OC
H/471/Cx
I/870/OC
JUW-36/Cx
K/UW-31Cx
$L_1$/440/Bu
$L_2$/434/Bu
$L_3$/404/Bu

6. A substance for the detection of antibodies to predetermined strains of <u>C. trachomatis</u> comprising an immunologically inert substrate having stabilized reticulated body of <u>C. trachomatis</u> fixed thereto.

7. A substance according to claim 6 characterized in that the reticulated body was previously stabilized with formaldehyde.

8. A substance according to claim 6 or claim 7 characterized in that the reticulated body antigen is derived from at least one strain having an antigenic reaction to antibodies to at least one of the strains designated in WHO reference as:

ASA-1/OT

B/TW1

C/TW8/OT

D/IC-Ca180N

E/Bour/OT

F/IC-Cal-30N

G/392/OC

H/471/Cx

I/870/OC

JUW-36/Cx

K/UW-31Cx

$L_1$/440/Bu

$L_2$/434/Bu

$L_3$/404/Bu

0017460

# EUROPEAN SEARCH REPORT

European Patent Office

Application number

EP 80301003.2

## DOCUMENTS CONSIDERED TO BE RELEVANT

| Category | Citation of document with indication, where appropriate, of relevant passages | Relevant to claim |
|---|---|---|
| D | <u>US - A - 4 118 469</u> (CALDWELL et al.)<br><br>+ Totality +<br><br>---- | 1 |

**CLASSIFICATION OF THE APPLICATION (Int Cl. ³)**

G 01 N 33/54//

(C 12 Q 1/70)

**TECHNICAL FIELDS SEARCHED (Int.Cl. ³)**

G 01 N

C 12 Q

A 61 B

**CATEGORY OF CITED DOCUMENTS**

X: particularly relevant

A: technological background

O: non-written disclosure

P: intermediate document

T: theory or principle underlying the invention

E: conflicting application

D: document cited in the application

L: citation for other reasons

&: member of the same patent family, corresponding document

X | The present search report has been drawn up for all claims

| Place of search | Date of completion of the search | Examiner |
|---|---|---|
| VIENNA | 20-06-1980 | SCHNASS |

EPO Form 1503.1 06.78